(19) Europäisches Patentamt
European Patent Office
Office européen des brevets

(11) Publication number: 0 033 183
B1

(12) **EUROPEAN PATENT SPECIFICATION**

(45) Date of publication of patent specification: **04.07.84**

(51) Int. Cl.³: **C 07 D 213/81**, C 07 D 213/82, C 07 D 213/83, C 07 D 213/78, C 07 D 213/57

(21) Application number: **81200281.4**

(22) Date of filing: **30.07.79**

(60) Publication number of the earlier application in accordance with Art. 76 EPC: **0008145**

(54) **Phenyliminomethyl-3-pyridine derivatives, their preparation and use.**

(30) Priority: **08.08.78 GB 3252378**

(43) Date of publication of application: **05.08.81 Bulletin 81/31**

(45) Publication of the grant of the patent: **04.07.84 Bulletin 84/27**

(84) Designated Contracting States: **AT BE CH DE FR GB IT LU NL**

(56) References cited: **US - A - 3 697 505**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Ten Haken, Pieter**
**Konkelboet The Street Eastling**
**Near Faversham Kent (GB)**
Inventor: **Webb, Shirley Beatrice**
**17 North Street Ashford Road**
**Sheldwich Near Faversham Kent (GB)**
Inventor: **Smith, Graham Clifford**
**3 Beaconsfield Road**
**Sittingbourne Kent (GB)**

(74) Representative: **Hunter, Keith Roger Ian et al,**
**4 York Road**
**London SE1 7NA (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 033 183 B1

Courier Press, Leamington Spa, England.

**Description**

The present invention relates to novel phenylimino-C-(halo)-methyl-3-pyridine derivatives, to a process for their preparation and to their use as intermediates.

It has been found that certain phenyliminomethylpyridine deriviatives show activity as fungicides and plant growth regulants. These derivatives are described in our copending application, EP—A—0 008 145, out of which this application is divided. The present invention concerns phenylimino-C-(chloro)methyl-3-pyridine derivatives which may be used in a process for the preparation of some of those phenylimino methylpyridine derivatives.

Accordingly the present invention provides phenylimino-C-(chloro)methyl-3-pyridine derivatives of formula (I)

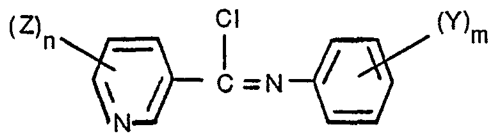

(I)

wherein each Y and Z independently are halogen atoms, alkyl, alkoxy or nitro groups, and m and n are each 0, 1 or 2, whereby the same or different groups Y may be present when m equals 2 and the same or different groups Z may be present when n equals 2.

The pyridyl group may be non-substituted or substituted, suitably with one or more halogen atoms, such as fluorine, chlorine or bromine atoms, alkyl groups, such as methyl or ethyl groups, alkoxy groups, such as methoxy, ethoxy or isopropoxy groups or nitro groups. Halogen atoms, in particular chlorine atoms, are preferred substituents.

The phenyl group in the compounds of formula I may also be non-substituted or substituted with 1 or 2 groups; these groups may suitably be those recommended as substituents of the pyridyl group. Preferably Y in formula I represents a halogen atom, particularly a chlorine atom.

A particularly preferred compound of the invention is 4′-phenylimino-C-(chloro)methyl-3-pyridine, because of the fungicidal activity of the end products which may be prepared from it.

The phenylimino-C-(chloro)methyl-3-pyridine derivatives of the present invention may be prepared by reacting an optionally substituted nicotinic anilide with thionyl chloride.

A further aspect of the present invention comprises the use of the derivatives of formula (I) above as intermediates, and in particular as intermediates in the preparation of certain of the biologically active phenyliminomethyl pyridine derivatives referred to above. Thus the phenylimino-C-(chloro)-methyl-3-pyridine derivatives may be used to prepare phenyliminomethyl-3-pyridine derivatives of the formula (II):

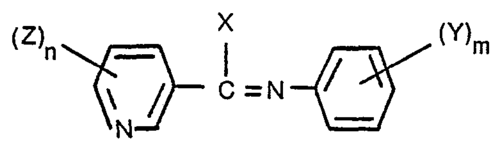

(II)

wherein X represents a cyano group, a non-substituted or a halo- or alkoxy-substituted alkoxy group, a cycloalkyloxy group, an alkylthio, carboxymethylthio, 2-chlorophenylthio, 4-chlorophenylthio, 4-bromophenylthio, 2,4-dichlorophenylthio, a group —NRR′, in which R is a hydrogen atom or an alkyl group, R′ is an alkyl, a phenyl or a cycloalkyl group and when R is hydrogen R′ can be p-fluorophenyl, benzyl, diisopropylaminoethyl, methoxyethyl or hydroxyethyl or R and R′ together with the interjacent nitrogen atom may form a heterocyclic ring, chosen from triazole, pyrrolidine, morpholine, piperidine or 3,4-dimethylpiperidine and Y and Z independently are halogen atoms, alkyl, alkoxy or nitro groups and m and n are each 0, 1 or 2, whereby the same or different groups Y may be present when m equals 2 and the same or different groups Z may be present when n equals 2. The derivatives of formula (II) above are described and claimed together with their use as fungicides and plant growth regulants, and their preparation from optionally substituted nicotinic anilides in our EP—8745 referred to above.

The invention is further illustrated by the following examples in which compounds of the invention are used as intermediates in the preparation of the compounds of formula (II) above.

Example 1

Preparation of 4′-chlorophenylimino-C-(cyano)methyl-3-pyridine

A stirred mixture of nicotinic-4′-chloroanilide (4.65 g, 0.02 mol) and thionyl chloride (20 ml) was heated under reflux for 2 hours. Excess thionyl chloride was removed *in vacuo,* and the residue treated

with dry pyridine (50 ml). To the stirred solution was added dry cuprous cyanide (3.58 g, 0.04 mole). After 15 min., pyridine was removed *in vacuo,* and the residue was extracted with warm acetone. Evaporation of the solvent from the extract gave the 4′-chlorophenylimino-C-(cyano)methyl-3-pyridine in 60% yield as a yellow crystalline solid, melting point 73—75°C.

Analysis:

| | | | |
|---|---|---|---|
| Calculated for $C_{13}H_8N_3Cl$: | C 64.6; | H 3.31; | N 17.39% |
| Found | C 63.7; | H 3.3; | N 17.0% |

Example 2

Preparation of 4′-chlorophenylimino-C-(isopropoxy)methyl-3-pyridine

A stirred mixture of nicotinic-4′-chloroanilide (7 g, 0.03 mole) and thionyl chloride (22.5 ml) was heated under reflux for 2 hours. Excess thionyl chloride was removed *in vacuo,* and the residue suspended in dry dimethoxyethane (75 ml). A solution of sodium (2.43 g, 0.105 mole) in dry isopropanol (100 ml) was added all at once, and the mixture was stirred for 1 h at room temperature and then heated under reflux for 16 hours. Solvents were removed *in vacuo,* and the residue treated with ether; the ethereal solution was washed with water (2 ×) and dried over $MgSO_4$. After removal of the solvent the residue was subjected to column chromatography on silica gel, eluting with $Et_2O$/hexane (1:1). The 4′-chlorophenylimino-C-(isopropoxy)methyl-3-pyridine was obtained, after recrystallization from 60/80 petroleum spirit, in 27.5% yield as a colourless crystalline solid, melting point 71—72.5°C.

Analysis:

| | | | |
|---|---|---|---|
| Calculated for $C_{15}H_{15}N_2OCl$: | C 65.6; | H 5.5; | N 10.2% |
| Found: | C 65.4; | H 5.8; | N 10.0% |

Example 3

Preparation of 4′-chlorophenylimino-C-(n-propoxy)methyl-3-pyridine

A stirred mixture of nicotinic-4′-chloroanilide (7 g, 0.03 mole) and thionyl chloride (22.5 ml) was heated under reflux for 2 hours. Excess thionyl chloride was removed *in vacuo*, and the residue suspended in dry dimethoxyethane (75 ml). A solution of sodium (2.42 g, 0.105 mole) in dry *n*-propanol (100 ml) was added all at once, and the mixture stirred for 1 h at room temperature, and then heated under reflux for 16 hours. Solvents were removed *in vacuo*, and the residue treated with chloroform, the chloroform solution was washed with water (2 ×) and dried over $MgSO_4$. After removal of the solvent, the residue was subjected to column chromatography on silica gel, eluting with chloroform/ethyl acetate (4:1). The 4′-chlorophenylimino-C-(*n*-propoxy)methyl-3-pyridine was obtained in 58.5% yield as an oil.

Analysis:

| | | | |
|---|---|---|---|
| Calculated for $C_{15}H_{15}N_2OCl$: | C 65.6; | H 5.5; | N 10.2% |
| Found: | C 66.4; | H 5.8; | N 10.1% |

Example 4

Preparation of 4′-chlorophenylimino-C-(n-butoxy)methyl-3-pyridine

A stirred mixture of nicotinic-4′-chloroanilide (7 g, 0.03 mole) and thionyl chloride (22.5 ml) was heated under reflux for 2 hours. Excess thionyl chloride was removed *in vacuo,* and the residue suspended in dry dimethoxyethane (75 ml). A solution of sodium (2.42 g, 0.015 mole) in dry *n*-butanol (100 ml) was added all at once, and the mixture was stirred at room temperature for 1 h, and then heated under reflux for 16 hours. Solvents were removed *in vacuo*, and the residue treated with chloroform; the chloroform solution was washed with water (2 ×) and dried over $MgSO_4$. After removal of the solvent, the residue was subjectd to column chromatography on silica gel, eluting with ether/hexane (1:1). The 4′-chlorophenylimino-C-(*n*-botoxy)methyl-3-pyridine was obtained, after recrystallization from 40/60 petroleum spirit, in 46% yield as colourless crystals, melting point 53—54°C.

Analysis:

| | | | |
|---|---|---|---|
| Calculated for $C_{16}H_{17}N_2OCl$: | C 66.6; | H 5.9; | N 9.7% |
| Found: | C 66.5; | H 6.2; | N 9.9% |

Example 5

Preparation of 4′-chlorophenylimino-C-(cyclohexyloxy)methyl-3-pyridine

A mixture of nicotinic-4′-chloroanilide (7 g, 0.03 mole) and thionyl chloride (22.5 ml) was stirred and heated under reflux for 2 hours. Excess thionyl chloride was removed *in vacuo,* and the residue treated with dry pyridine (100 ml). To the stirred solution was added redistilled cyclohexanol (3.15 g, 5% excess), and subsequently the mixture was heated in an oil bath at 100°C for 6½ hours. After

removal of the solvent in vacuo, the residue was treated with ether; the ethereal solution was washed with water (2 x), and dried over $MgSO_4$. After removal of the solvent, the residue was subjected to column chromatography on silica gel, eluting with ether/hexane (1:1). The 4'-chlorophenylimino-C-(cyclohexyloxy)methyl-3-pyridine was obtained in 32% yield as a viscous oil.

Analysis:

| | | | |
|---|---|---|---|
| Calculated for $C_{18}H_{19}N_2OCl$: | C 68.7; | H 6.0; | N 8.9% |
| Found: | C 67.8; | H 6.2; | N 8.7% |

Example 6

Preparation of 4'-chlorophenylimino-C-(tert.-butylthio)methyl-3-pyridine

A stirred mixture of nicotinic-4'-chloroanilide (7 g, 0.03 mole) and thionyl chloride (22.5 ml) was heated under reflux for 2 hours. Excess thionyl chloride was removed *in vacuo,* and the residue treated with dry pyridine (100 ml). Tert.-butyl mercaptan (5.4 g, 0.06 mole) was added, and the mixture stirred and heated in an oil bath at 100—110°C for 16 hours. Solvent and volatiles were removed *in vacuo,* and the residue was treated with ether; the ethereal solution was washed with water (3 x), and dried over $MgSO_4$. After removal of solvent, the residue was subjected to column chromatography on silica gel, eluting with $Et_2O$/hexane (1:1). The 4'-chlorophenylimino-C-(*tert.*-butylthio)methyl-3-pyridine was obtained in 24% yield as a bright yellow crystalline solid, melting point 85—87°C.

Analysis:

| | | | |
|---|---|---|---|
| Calculated for $C_{16}H_{17}N_2SCl$: | C 63.05; | H 5.58; | N 9.20% |
| Found: | C 62.8; | H 5.7; | N 9.1% |

Example 7

Preparation of 4'-chlorophenylimino-C-(carboxymethylthio)methyl-3-pyridine

A stirred mixture of nicotinic-4'-chloroanilide (4.65 g, 0.02 mole) and thionyl chloride (20 ml) was heated under reflux for 2 hours. Excess thionyl chloride was removed *in vacuo,* and the residue treated with dry pyridine (50 ml). To this solution was added ethyl 2-mercaptoacetate (3.0 g, 0.025 mole) and the mixture was shaken for 3 hours at ambient temperature. Solvent was removed *in vacuo,* and the residue was treated with ether; the ethereal solution was washed with water and dried over $MgSO_4$. The crude ester, obtained by evaporation of the solvent, was treated with NaOH (1.2 g 0.03 mole) in $H_2O$ (10 ml) and methanol (35 ml), and the mixture was left for $2\frac{1}{2}$ hours at room temperature. Solvent was removed *in vacuo,* the residue treated with water (50 ml) and the aqueous solution extracted twice with ether. The aqueous phase was neutralized with acetic acid, to give an oil, which solidified. This was filtered off, and recrystallized from toluene, to give the product in 46% yield as an orange-brown solid, melting point 142—4°C.

Analysis:

| | | | |
|---|---|---|---|
| Calculated for $C_{14}H_{11}N_2SO_2Cl$: | C 54.8; | H 3.6; | N 9.1% |
| Found: | C 54.8; | H 3.5; | N 8.9% |

Example 8

Preparation of 4'-chlorophenylimino-C-(n-butylamino)methyl-3-pyridine

A stirred mixture of nicotinic-43-chloroanilide (7 g 0.03 mole) and thionyl chloride (22.5 ml) was heated under reflux for 2 hours. Excess thionyl chloride was removed *in vacuo,* and the residue suspended in dry dimethoxyethane (75 ml). *n*-Butylamine (10.8 g, 0.15 mole) was added, and the mixture was stirred at ambinent temperature for 24 hours. Solvent was removed *in vacuo,* and the residue treated with ether; the ethereal solution was washed with water (2 x) and dried over $MgSO_4$. After removal of solvent, the residue was subjected to column chroamtography on silica gel, eluting with $Et_2O$/hexane (2:1). The 4'-chlorophenylimino-C-(*n*-butylamino)methyl-3-pyridine thus obtained after recrystallization from 60/80 petroleum spirit, had a melting point of 65—67°C. The yield was 50%.

Analysis:

| | | | |
|---|---|---|---|
| Calculated for $C_{16}H_{18}N_3Cl$: | C 66.78; | H 6.26; | N 14.61% |
| Found: | C 66.7; | H 6.5; | N 14.4% |

Example 9

Preparation of 4'-chlorophenylimino-C-(di-n-butylamino)methyl-3-pyridine

A mixture of nicotinic-4'-chloroanilide (7 g, 0.03 mole) and thionyl chloride (22.5 ml) was stirred and heated under reflux for 2 hours. Excess thionyl chloride was removed *in vacuo,* and the residue suspended in dry dimethoxyethane (150 ml). Di-*n*-butylamine (19.4 g, 0.15 mole) was added, and the mixture was stirred for 2 hours at ambient temperature. The solvent was removed *in vacuo,* and the residue treated with ether; the ethereal solution was washed with water (2 x) and dried over $MgSO_4$.

After removal of the solvent, the residue was subjected to column chromatography on silica gel, eluting with ether/hexane (1:1). The 4′-chlorophenylimino-C-(di-*n*-butylamino)-methyl-3-pyridine was obtained in 27% yield as a viscous oil.

Analysis:

| | | | |
|---|---|---|---|
| Calculated for $C_{20}H_{26}N_3Cl$: | C69.9; | H 7.6; | N 12.2% |
| Found: | C 69.5; | H 7.2; | N 11.8% |

Example 10

Preparation of 4′-chlorophenylimino-C-(N-piperidinyl)methyl-3-pyridine

A stirred mixture of nicotinic-4′-chloroanilide (7 g, 0.03 mole) and thionyl chloride (22.5 ml) was heated under reflux for 2 hours. Excess thionyl chloride was removed *in vacuo,* and the residue suspended in dry dimethoxyethane (100 ml). Piperidine (9 g, 0.105 mole) was added, and the mixture stirred at ambient temperature for 16 hours. Solvent was removed *in vacuo,* and the residue treated with ether; the ethereal solution was washed with water (2 ×) and dried over $MgSO_4$. After removal of solvent, the residue was subjected to column chromatography on silica gel, eluting with $Et_2O$/hexane (1:1). The 4′-chlorophenylimino-C-(N-piperidinyl)methyl-3-pyridine was obtained in 11% yield as a viscous oil.

Analysis:

| | | | |
|---|---|---|---|
| Calculated for $C_{17}H_{18}N_3Cl$: | C 68.11; | H 6.01; | N 14.02% |
| Found: | C 68.3; | H 6.5; | N 13.0% |

Examples 11—71

In an analogous manner as described in the previous Examples the following compounds were prepared:

| Example No. | Compound | Analysis | C | H | N |
|---|---|---|---|---|---|
| 11 | 4′-chlorophenylimino-C-(ethoxy)methyl-3-pyridine | Calc. for $C_{14}H_{13}N_2OCl$: | 64.50 | 5.0 | 10.8 |
| | | Found: | 64.9 | 5.1 | 10.5 |
| 12 | 4′-chlorophenylimino-C-(p-fluoroanilino)methyl-3-pyridine | Calc. for $C_{18}H_{13}N_3ClF$: | 66.4 | 4.0 | 12.9 |
| | | Found: | 66.3 | 3.9 | 13.0 |
| 13 | 4′-chlorophenylimino-C-(methoxy)methyl-3-pyridine | Calc. for $C_{13}H_{11}N_2OCl$: | 63.3 | 4.5 | 11.4 |
| | | Found: | 63.6 | 4.6 | 11.7 |
| 14 | 4′-chlorophenylimino-C-(triazolyl)methyl-3-pyridine | Calc. for $C_{14}H_{10}N_5Cl$: | 59.3 | 3.5 | 24.7 |
| | | Found: | 59.4 | 3.4 | 23.9 |
| 15 | 4′-chlorophenylimino-C-(2-butoxy)methyl-3-pyridine | Calc. for $C_{16}H_{17}N_2OCl$: | 66.6 | 5.9 | 9.7 |
| | | Found: | 67.0 | 6.2 | 10.0 |
| 16 | 4′-chlorophenylimino-C-(isobutoxy)methyl-3-pyridine | Calc. for $C_{16}H_{17}N_2OCl$: | 66.6 | 5.9 | 9.7 |
| | | Found: | 66.2 | 6.2 | 9.6 |
| 17 | 4′-chlorophenylimino-C-(n-octyloxy)methyl-3-pyridine | Calc. for $C_{20}H_{25}N_2OCl$: | 69.7 | 7.3 | 8.1 |
| | | Found: | 69.7 | 7.6 | 8.4 |
| 18 | 4′-chlorophenylimino-C-(n-dodecyloxy)methyl-3-pyridine | Calc. for $C_{24}H_{33}N_2OCl$: | 71.9 | 8.2 | 7.0 |
| | | Found: | 71.8 | 8.2 | 7.0 |
| 19 | 4′-chlorophenylimino-C-(isopropylthio)methyl-3-pyridine | Calc. for $C_{15}H_{15}N_2SCl$: | 61.96 | 5.2 | 9.6 |
| | | Found: | 61.8 | 5.2 | 9.6 |
| 20 | 4′-chlorophenylimino-C-(p-chlorophenylthio)methyl-3-pyridine | Calc. for $C_{18}H_{12}N_2SCl_2$: | 60.2 | 3.3 | 7.8 |
| | | Found: | 60.3 | 3.3 | 7.8 |
| 21 | 4′-chlorophenylimino-C-(di-n-propylamino)methyl-3-pyridine | Calc. for $C_{18}H_{22}N_3Cl$: | 68.5 | 7.0 | 13.3 |
| | | Found: | 68.1 | 7.6 | 12.5 |
| 22 | 4′-chlorophenylimino-C-(n-nonylamino)methyl-3-pyridine | Calc. for $C_{21}H_{28}N_3Cl$: | 70.5 | 7.8 | 11.8 |
| | | Found: | 70.5 | 7.9 | 11.5 |
| 23 | 4′-chlorophenylimino-C-(methoxyethoxy)methyl-3-pyridine | Calc. for $C_{15}H_{15}N_2O_2Cl$: | 62.0 | 5.2 | 9.6 |
| | | Found: | 62.3 | 5.5 | 9.5 |

| Example No. | Compound | Analysis | C | H | N |
|---|---|---|---|---|---|
| 24 | 2-chloro-4′-chlorophenylimino-C-(isopropoxy)methyl-3-pyridine | Calc. for $C_{15}H_{14}N_2OCl_2$: | 58.3 | 4.5 | 9.1 |
| | | Found: | 58.0 | 4.4 | 8.9 |
| 25 | 4′-fluorophenylimino-C-(isopropoxy)methyl-3-pyridine | Calc. for $C_{15}H_{15}N_2OF$: | 69.8 | 5.8 | 10.9 |
| | | Found: | 69.7 | 5.8 | 10.7 |
| 26 | 3′,5′-dichlorophenylimino-C-(isopropoxy)methyl-3-pyridine | Calc. for $C_{15}H_{14}N_2OCl$: | 58.3 | 4.5 | 9.1 |
| | | Found: | 58.6 | 4.6 | 9.0 |
| 27 | 3′,5′-dichlorophenylimino-C-(isopropylthio)methyl-3-pyridine | Calc. for $C_{15}H_{14}N_2SCl_2$: | 55.4 | 4.3 | 8.6 |
| | | Found: | 55.7 | 4.4 | 8.6 |
| 28 | 2′,6′-dimethylphenylimino-C-(isopropoxy)methyl-3-pyridine | Calc. for $C_{17}H_{20}N_2O$: | 76.1 | 7.5 | 10.5 |
| | | Found: | 77.0 | 7.7 | 10.2 |
| 29 | 4′-fluorophenylimino-C-(isopropylthio)methyl-3-pyridine | Calc. for $C_{15}H_{15}N_2SF$: | 65.7 | 5.5 | 10.2 |
| | | Found: | 66.0 | 5.7 | 10.4 |
| 30 | 3′,4′-dichlorophenylimino-C-(isopropoxy)methyl-3-pyridine | Calc. for $C_{15}H_{14}N_2OCl_2$: | 58.3 | 4.5 | 9.1 |
| | | Found: | 58.1 | 4.4 | 8.8 |
| 31 | Phenylimino-C-(isopropoxy)methyl-3-pyridine | Calc. for $C_{15}H_{16}N_2O$: | 75.0 | 6.7 | 11.7 |
| | | Found: | 75.5 | 7.3 | 11.2 |
| 32 | 3′,4′-dichlorophenylimino-C-(isopropylthio)methyl-3-pyridine | Calc. for $C_{15}H_{14}N_2SCl_2$: | 55.4 | 4.3 | 8.6 |
| | | Found: | 56.1 | 4.5 | 8.7 |
| 33 | Phenylimino-C-(isopropylthio)methyl-3-pyridine | Calc. for $C_{15}H_{16}N_2S$: | 70.3 | 6.2 | 11.0 |
| | | Found: | 71.7 | 6.7 | 11.1 |
| 34 | 4′-bromophenylimino-C-(isopropoxy)methyl-3-pyridine | Calc. for $C_{15}H_{15}N_2OBr$: | 56.4 | 4.7 | 8.8 |
| | | Found: | 55.6 | 4.8 | 8.6 |
| 35 | 4′-bromophenylimino-C-(isopropylthio)methyl-3-pyridine | Calc. for $C_{15}H_{15}N_2SBr$: | 53.7 | 4.5 | 8.4 |
| | | Found: | 53.5 | 4.5 | 8.3 |
| 36 | 2′,4′-dichlorophenylimino-C-(isopropoxy)methyl-3-pyridine | Calc. for $C_{15}H_{14}N_2OCl_2$: | 58.3 | 4.5 | 9.1 |
| | | Found: | 58.0 | 4.5 | 8.9 |
| 37 | 2-isopropoxy-4′-chlorophenylimino-C-(isopropoxy)methyl-5-pyridine | Calc. for $C_{18}H_{21}N_2O_2Cl$: | 65.0 | 6.3 | 8.4 |
| | | Found: | 64.3 | 6.0 | 7.9 |

| Example No. | Compound | Analysis | C | H | N |
|---|---|---|---|---|---|
| 38 | 3′-chloro-4′-fluorophenylimino-C-(isopropylthio)methyl-3-pyridine | Calc. for $C_{15}H_{14}N_2SClF$: | 58.4 | 4.5 | 9.1 |
| | | Found: | 58.6 | 4.9 | 8.9 |
| 39 | 2′,4′-dichlorophenylimino-C-(isopropylthio)methyl-3-pyridine | Calc. for $C_{15}H_{14}N_2SCl_2$: | 55.4 | 4.3 | 8.6 |
| | | Found: | 56.1 | 4.6 | 8.5 |
| 40 | 2-chloro-4′-chlorophenylimino-C-(isopropylthio)methyl-5-pyridine | Calc. for $C_{15}H_{14}N_2SCl_2$: | 55.4 | 4.3 | 8.6 |
| | | Found: | 55.5 | 4.2 | 8.4 |
| 41 | 4′-chlorophenylimino-C-(diisopropylaminoethylamino)methyl-3-pyridine | Calc. for $C_{20}H_{27}N_4Cl$: | 67.0 | 7.5 | 15.6 |
| | | Found: | 67.0 | 7.5 | 15.5 |
| 42 | 4′-chlorophenylimino-C-(di-(3-methylbutyl)amino)methyl-3-pyridine | Calc. for $C_{22}H_{30}N_3Cl$: | 71.1 | 8.1 | 11.3 |
| | | Found: | 71.4 | 8.4 | 11.1 |
| 43 | 4′-chlorophenylimino-C-(methoxyethylamino)methyl-3-pyridine | Calc. for $C_{15}H_{16}N_3OCl$: | 62.2 | 5.5 | 14.5 |
| | | Found: | 62.2 | 5.5 | 14.6 |
| 44 | 4′-chlorophenylimino-C-(*n*-hexadecylamino)methyl-3-pyridine | Calc. for $C_{28}H_{42}N_3Cl$: | 73.8 | 9.2 | 9.2 |
| | | Found: | 74.1 | 9.9 | 8.9 |
| 45 | 4′-chlorophenylimino-C-(hydroxyethylamino)methyl-3-pyridine | Calc. for $C_{14}H_{14}N_3OCl$: | 61.0 | 5.1 | 15.3 |
| | | Found: | 61.0 | 5.2 | 15.2 |
| 46 | 4′-chlorophenylimino-C-(N-pyrolidinyl)methyl-3-pyridine | Calc. for $C_{16}H_{16}N_3Cl$: | 67.3 | 5.6 | 14.7 |
| | | Found: | 67.4 | 5.7 | 14.8 |
| 47 | 4′-chlorophenylimino-C-(benzylamino)methyl-3-pyridine | Calc. for $C_{19}H_{16}N_3Cl$: | 70.9 | 5.0 | 13.1 |
| | | Found: | 70.6 | 5.0 | 12.9 |
| 48 | 2-chloro-4′-chlorophenylimino-C-(isopropoxy)methyl-5-pyridine | Calc. for $C_{15}H_{14}N_2OCl_2$: | 58.3 | 4.5 | 9.1 |
| | | Found: | 58.3 | 4.6 | 9.0 |
| 49 | 3′-chlorophenylimino-C-(isopropoxy)methyl-3-pyridine | Calc. for $C_{15}H_{15}N_2OCl$: | 65.6 | 5.5 | 9.8 |
| | | Found: | 66.6 | 5.9 | 10.2 |
| 50 | 3′-chloro-4′-fluorophenylimino-C-(isopropoxy)methyl-3-pyridine | Calc. for $C_{15}H_{14}N_2OClF$: | 61.5 | 4.8 | 9.6 |
| | | Found: | 61.6 | 4.9 | 9.4 |
| 51 | 4′-chlorophenylimino-C-(N-morpholinyl)methyl-3-pyridine | Calc. for $C_{16}H_{12}N_3OCl$: | 63.6 | 5.3 | 13.9 |
| | | Found: | 63.8 | 5.2 | 14.0 |

| Example No. | Compound | Analysis | C | H | N |
|---|---|---|---|---|---|
| 52 | 4′-chlorophenylimino-C -(di-(2-methylpropyl)amino)methyl-3-pyridine | Calc. for $C_{20}H_{26}N_3Cl$: | 69.9 | 7.6 | 12.2 |
| | | Found: | 71.2 | 8.1 | 12.3 |
| 53 | 4′-chlorophenylimino-C-(3,5-dimethylpiperidin-1-yl)methyl-3-pyridine | Calc. for $C_{19}H_{22}N_3Cl$: | 69.62 | 6.72 | 12.82 |
| | | Found: | 69.5 | 6.9 | 12.9 |
| 54 | 2′,3′-dichlorophenylimino-C-(isopropoxy)methyl-3-pyridine | Calc. for $C_{15}H_{14}N_2OCl_2$: | 58.25 | 4.53 | 9.06 |
| | | Found: | 57.9 | 4.6 | 8.9 |
| 55 | 2′-chlorophenylimino-C-(n-butoxy)methyl-3-pyridine | Calc. for $C_{16}H_{17}N_2OCl$: | 66.6 | 5.9 | 9.7 |
| | | Found: | 66.5 | 6.1 | 9.6 |
| 56 | 4′-methylphenylimino-C-(n-butoxy)methyl-3-pyridine | Calc. for $C_{17}H_{20}N_2O$: | 76.1 | 7.5 | 10.4 |
| | | Found: | 76.5 | 7.7 | 10.4 |
| 57 | 3′-methylphenylimino-C-(n-butoxy)methyl-3-pyridine | Calc. for $C_{17}H_{20}N_2O$: | 76.1 | 7.5 | 10.4 |
| | | Found: | 75.3 | 7.7 | 10.4 |
| 58 | 2′-methylphenylimino-C-(n-butoxy)methyl-3-pyridine | Calc. for $C_{17}H_{20}N_2O$: | 76.1 | 7.5 | 10.4 |
| | | Found: | 76.1 | 8.0 | 10.0 |
| 59 | 2′,3′-dichlorophenylimino-C-(n-butoxy)methyl-3-pyridine | Calc. for $C_{16}H_{16}N_2OCl_2$: | 59.63 | 4.97 | 8.7 |
| | | Found: | 58.5 | 5.0 | 8.4 |
| 60 | 4′-methylphenylimino-C-(isopropylthio)methyl-3-pyridine | Calc. for $C_{16}H_{18}N_2S$: | 71.1 | 6.67 | 10.37 |
| | | Found: | 70.7 | 6.9 | 10.4 |
| 61 | 2′,5′-dichlorophenylimino-C-(isopropylthio)methyl-3-pyridine | Calc. for $C_{15}H_{14}N_2SCl_2$: | 55.4 | 4.31 | 8.62 |
| | | Found: | 55.2 | 4.4 | 8.5 |
| 62 | 2′,6′-dichlorophenylimino-C-(n-butoxy)methyl-3-pyridine | Calc. for $C_{16}H_{16}N_2OCl_2$: | 59.44 | 4.95 | 8.7 |
| | | Found: | 59.7 | 5.2 | 8.6 |
| 63 | 2′,6′-dichlorophenylimino-C-(isopropylthio)methyl-3-pyridine | Calc. for $C_{15}H_{14}N_2SCl_2$: | 55.4 | 4.31 | 8.62 |
| | | Found: | 55.0 | 4.1 | 9.3 |
| 64 | 23-chloro-4′-methylphenylimino-C-(n-butoxy)methyl-3-pyridine | Calc. for $C_{17}H_{19}N_2OCl$: | 67.44 | 6.28 | 9.26 |
| | | Found: | 67.5 | 6.5 | 9.6 |
| 65 | 2′-chloro-4′-methylphenylimino-C-(isopropylthio)methyl-3-pyridine | Calc. for $C_{16}H_{17}N_2SCl$: | 63.05 | 5.58 | 9.2 |
| | | Found: | 63.4 | 5.9 | 9.2 |

| Example No. | Compound | Analysis | C | H | N |
|---|---|---|---|---|---|
| 66 | 3′-chloro-4′-methylphenylimino-C-(n-butoxy)methyl-3-pyridine | alc. for $C_{17}H_{19}N_2OCl$:<br>Found: | 67.44<br>67.4 | 6.28<br>6.4 | 9.26<br>9.1 |
| 67 | 3′-chloro-4′-methylphenylimino-C-(isopropylthio)methyl-3-pyridine | Calc. for $C_{16}H_{17}N_2SCl$:<br>Found: | 63.05<br>63.1 | 5.58<br>5.8 | 9.2<br>9.0 |
| 68 | 2′-methyl-4′-chlorophenylimino-C-(n-butoxy)methyl-3-pyridine | Calc. for $C_{17}H_{19}N_2OCl$:<br>Found: | 67.44<br>67.3 | 6.28<br>6.5 | 9.26<br>9.0 |
| 69 | 2′-methyl-4′-chlorophenylimino-C-(isopropylthio)methyl-3-pyridine | Calc. for $C_{16}H_{17}N_2SCl$:<br>Found: | 63.05<br>63.1 | 5.58<br>5.8 | 9.2<br>9.0 |
| 70 | 2′-chloro-5′-methylphenylimino-C-(n-butoxy)methyl-3-pyridine | Calc. for $C_{17}H_{19}N_2OCl$:<br>Found: | 67.44<br>67.4 | 6.28<br>6.5 | 9.26<br>9.1 |
| 71 | 3′-methyl-4′-bromophenylimino-C-(n-butoxy)methyl-3-pyridine | Calc. for $C_{17}H_{19}N_2OBr$:<br>Found: | 58.81<br>58.5 | 5.48<br>5.6 | 8.07<br>7.8 |

**Claims for the Contracting States: BE CH DE FR GB IT LU NL**

1. A phenylimino-C-(chloro)methyl-3-pyridine derivative of the formula (I):

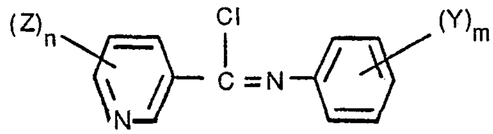

(I)

wherein Y and Z independently are halogen atoms, alkyl, alkoxy or nitro groups and m and n are each 0, 1 or 2, whereby the same or different groups Y may be present when m equals 2 and the same or different groups Z may be present when n equals 2.

2. The use of a derivative as claimed in claim 1 as an intermediate to prepare phenylimino methyl-3-pyridine derivatives of formula (II):

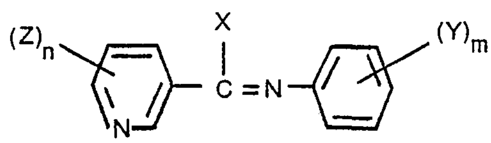

(II)

wherein X represents a cyano group, a non-substituted or a halo- or alkoxy-substituted alkoxy-group a cycloalkyloxy group, an alkylthio, carboxymethylthio, phenylthio, 2-chlorophenylthio, 4-chlorophenylthio, 4-bromophenylthio, 2,4-dichlorophenylthio, a group —NRR′, in which R is a hydrogen atom or an alkyl group and R′ is an alkyl, a phenyl or a cycloalkyl group and when R is hydrogen R′ can be p-fluorophenyl, benzyl, diisopropylamino ethyl, methoxyethyl or hydroxyethyl or R and R′ together with the interjacent nitrogen atom may form a heterocyclic ring chosen from triazole, pyrrolidine, morpholine, piperidine, or 3,4-dimethylpiperidine, and Y, Z, m and n have the meaning as in claim 1.

3. A process for the preparation of a derivative as claimed in claim 1 comprising reacting a nicotinic anilide of formula (III)

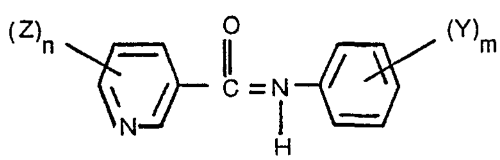

(III)

in which Y, Z n and m have the meaning as in claim 1, with thionylchloride.

**Claims for the Contracting State: AT**

1. The use of a phenylimino-C-(chloro)methyl-3-pyridine derivative of formula (I):

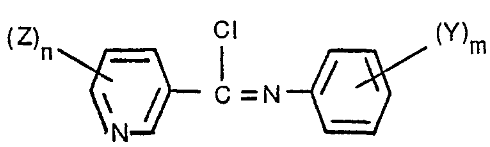

(I)

wherein Y and Z independently are halogen atoms, alkyl, alkoxy or nitro groups and m and n are each 0, 1 or 2, whereby the same or different groups Y may be present when m equals 2 and the same or different groups Z may be present when n equals 2, as an intermediate to prepare phenylimino methyl - 3 pyridine derivatives of formula (II):

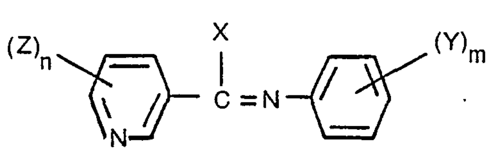

(II)

wherein X represents a cyano group, a non-substituted or a halo- or alkoxy-substituted alkoxy-group a cycloalkyloxy group, an alkylthio, carboxymethylthio, phenylthio, 2-chlorophenylthio, 4-chlorophenylthio, 4-bromophenylthio, 2,4-dichlorophenylthio, a group —NRR', in which R is a hydrogen atom or an alkyl group and R' is an alkyl, a phenyl or a cycloalkyl group and when R is hydrogen R' can be p-fluorophenyl, benzyl, diisopropylaminoethyl, methoxyethyl or hydroxyethyl or R and R' together with the interjacent nitrogen atom may form a heterocyclic ring chosen from triazole, pyrrolidine, morpholine, piperidine, or 3,4-dimethylpiperidine, and Y, Z, m and n have the above meaning.

2. A process for the preparation of a phenylimino-C-(chloro)methyl-3-pyridine derivative as defined in claim 1 comprising reacting a nicotinic anilide of formula (III):

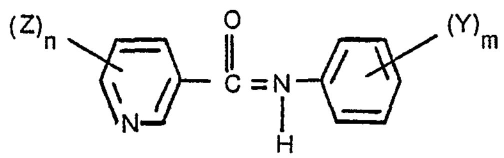

(III)

in which Y, Z, n and m have the meaning as in claim 1, with thionylchloride.

**Revendications pour les Etats contractants: BE CH DE FR GB IT LU NL**

1. Un dérivé de phénylimino-C-(chloro)méthyl-3-pyridine de formule (I):

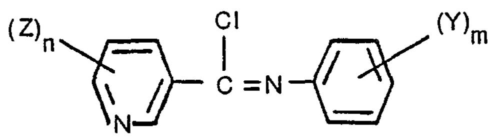

(I)

dans laquelle Y et Z indépendamment sont des atomes d'halogènes ou des groupes alcoyle, alcoxy ou nitro et m et n sont chacun 0, 1 ou 2, des groupes Y identiques ou différents pouvant être présents quand m = 2 et des groupes Z identiques ou différents pouvant être présents quand n = 2.

2. L'utilisation d'un dérivé selon la revendication 1 comme produit intermédiaire pour préparer des dérivés de phényliminométhyl-3-pyridine de formule (II):

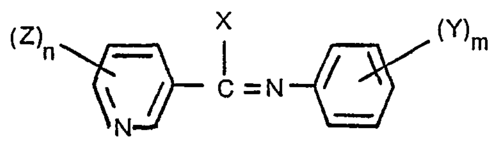

(II)

dans laquelle X représente un groupe cyano, un groupe alcoxy non-substitué ou halogéné ou alcoxylé, un groupe cycloalcoyloxy, un groupe alcoylthio, carboxyméthylthio, phénylthio, 2-chlorophénylthio, 4-chlorophénylthio, 4-bromophénylthio, 2,4-dichlorophénylthio, un groupe —NRR', dans lequel R est un atome d'hydrogène ou un groupe alcoyle et R' est un groupe alcoyle, phényle ou cycloalcoyle et quand R est de l'hydrogène R' peut être un groupe p-fluorophényle, benzyle, diisopropylaminoéthyle, méthoxyéthyle ou hydroxyéthyle ou R et R' en même temps que l'atome d'azote interjacent peuvent former un noyau hétérocyclique choisi parmi ceux de triazole, de pyrrolidine, de morpholine, de pipéridine ou de 3,4-diméthylpipéridine et Y, Z, m et n ont la même signification que dans la revendication 1.

3. Un procédé pour la préparation d'un dérivé tel que revendiqué dans la revendication 1, selon lequel on fait réagir un anilide nicotinique de formule (III):

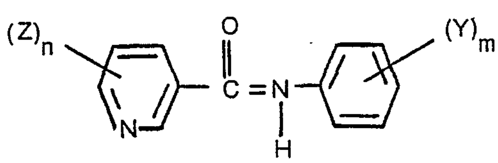

(III)

dans laquelle Y, Z, n et m ont la même signification que dans la revendication 1, avec le chlorure de thionyle.

**Revendications pour l'Etat contractant: AT**

1. L'utilisation d'un dérivé de phénylimino-C-(chloro)méthyl-3-pyridine de formule (I):

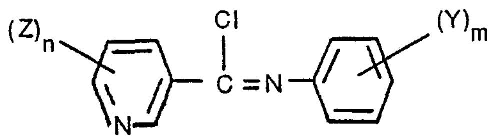

(I)

dans laquelle Y et Z indépendamment sont des atomes d'halogènes ou des groupes alcoyle, alcoxy ou nitro et m et n sont chacun 0, 1 ou 2, des groupes Y identiques ou différents pouvant être présents quand m = 2 et des groupes Z identiques ou différents pouvant être présents quand n = 2, comme produit intermédiaire pour la préparation de dérivés de phényliminométhyl-3-pyridine de formule (II):

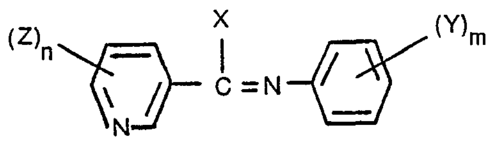

(II)

dans laquelle X représente un groupe cyano, un groupe alcoxy non-substitué ou halogéné ou alcoxylé, un groupe cycloalcoxy, un groupe alcoylthio, carboxyméthylthio, phénylthio, 2-chlorophénylthio, 4-chlorophénylthio, 4-bromophénylthio, 2,4-dichlorophenylthio, un groupe —NRR', dans lequel R est un atome d'hydrogène ou un groupe alcoyle et R' est un groupe alcoyle, phényle ou cycloalcoyle et quand R est de l'hydrogène, R' peut être un groupe p-fluorophényle, benzyle, diisopropylaminoéthyle, méthoxyéthyle ou hydroxyéthyle ou R et R' en même temps que l'atome d'azote interjacent peuvent former un noyau hétérocyclique choisi parmi ceux de triazole, de pyrrolidine, de morpholine, de pipéridine ou de 3,4-diméthylpipéridine, et Y, Z, m et n ont la signification ci-dessus.

2. Un procédé pour la préparation d'un dérivé de phénylimino-C-(chloro)méthyl-3-pyridine tel que défini dans la revendication 1, selon lequel on fait réagir un anilide nicotinique de formule (III):

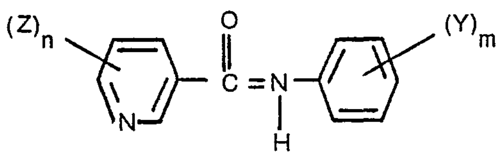

(III)

dans laquelle X, Z, n et m ont la même signification que dans la revendication 1, avec le chlorure de thionyle.

**Patentansprüche für die Vertragsstaaten: BE CH DE FR GB IT LU NL**

1. Ein Phenylimino-C-(chlor)methyl-3-pyridin-Derivat der Formel (I)

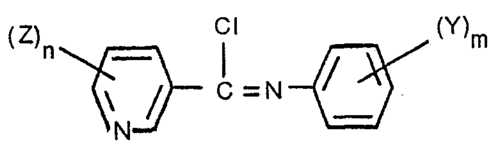

(I)

worin Y und Z unabhängig voneinander für Halogenatome, Alkyl-, Alkoxy- oder Nitrogruppen stehen und m und n jeweils 0, 1 oder 2 bedeuten, wobei gleiche oder unterschiedliche Gruppen Y vorliegen können, falls m den Wert 2 hat, und gleiche oder unterschiedliche Gruppen Z vorliegen können, wenn n den Wert 2 hat.

2. Die Verwendung eines Derivats wie in Anspruch 1 beansprucht als ein Zwischenprodukt zur Herstellung von Phenyliminomethyl-3-pyridin-Derivaten der Formel (II)

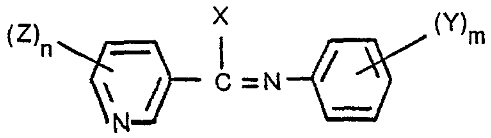

(II)

worin X für eine Cyanogruppe, eine unsubstituierte oder Halogen- oder Alkoxy-substituierte Alkoxygruppe, eine Cycloalkyloxygruppe, eine Alkylthiogruppe, Carboxymethylthiogruppe, Phenylthiogruppe, 2-Chlorphenylthiogruppe, 4-Chlorphenylthiogruppe, 4-Bromphenylthiogruppe, 2,4-Dichlorphenylthiogruppe oder eine Gruppe-NRR′ steht, worin R ein Wasserstoffatom oder eine Alkylgruppe ist und R′ eine Alkyl-, eine Phenyl- oder eine Cycloalkylgruppe bedeutet und, falls R für Wasserstoff steht, R′ p-Fluorphenyl, Benzyl, Diisopropylaminoethyl, Methoxyethyl oder Hydroxyethyl sein kann oder R und R′ gemeinsam mit dem dazwischenliegenden Stickstoffatom einen heterocyclischen Ring, ausgewählt unter Triazol, Pyrrolidin, Morpholin, Piperidin oder 3,4-Dimethylpiperidin, bilden können, und Y, Z, m und n die in Anspruch 1 angegebene Bedeutung haben.

3. Ein Verfahren zur Herstellung eines Derivates wie in Anspruch 1 beansprucht, umfassend ein Umsetzen eines Nicotinsäureanilids der Formel (III)

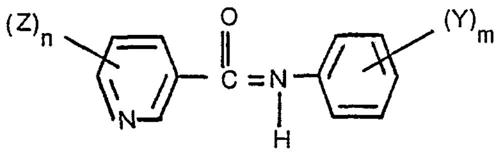

(III)

worin Y, Z, n und m die in Anspruch 1 angegebene Bedeutung aufweisen, mit Thionylchlorid.

**Patentansprüche für den Vertragsstaat: AT**

1. Die Verwendung eines Phenylimino-C-(chlor)methyl-3-pyridin-Derivats der Formel (I):

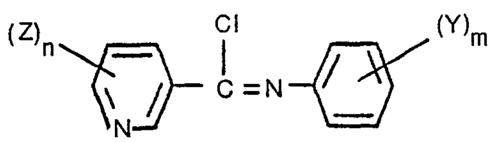

(I)

worin Y und Z unabhängig voneinander für Halogenatome, Alkyl-, Alkoxy- oder Nitrogruppen stehen, und m und n jeweils 0, 1 oder 2 bedeuten, wobei gleiche oder unterschiedlichen Gruppen Y vorliegen können, falls m den Wert 2 hat, und gleiche oder unterschiedliche gruppen Z vorliegen können, wenn n den Wert 2 hat, als ein Zwischenprodukt zur Herstellung von Phenyliminomethyl-3-pyridin-Derivaten der Formel (II):

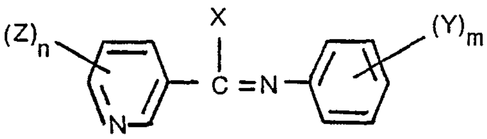

(II)

worin X für eine Cyanogruppe, eine unsubstituierte oder Halogen- oder Alkoxy-substituierte Alkoxygruppe, eine Cycloalkyloxygruppe, eine Alkylthiogruppe, Carboxymethylthiogruppe, Phenylthiogruppe, 2-Chlorphenylthiogruppe, 4-Chlorphenylthiogruppe, 4-Bromphenylthiogruppe, 2,4-Dichlorphenylthiogruppe oder eine Gruppe-NRR′ steht, worin R ein Wasserstoffatom oder eine Alkylgruppe ist und R′ eine Alkyl-, eine Phenyl- oder eine Cycloalkylgruppe bedeutet und, falls R für Wasserstoff steht, R′ p-Fluorphenyl, Benzyl, Diisopropylaminoethyl, Methoxyethyl oder Hydroxyethyl sein kann oder R und R′ gemeinsam mit dem dazwischenliegenden Stickstoffatom einen heterocyclischen Ring, ausgewählt unter Triazol, Pyrrolidin, Morpholin, Piperidin oder 3,4-Dimethylpiperidin, bilden können, unt Y, Z, m und n die vorstehend angegebene Bedeutung haben.

2. Verfahren zur Herstellung eines Phenylimino-C-(chlor)methyl-3-pyridin-Derivates, wie in Anspruch 1 definiert, umfassend ein Umsetzen eines Nicotinsäureanilids der Formel (III):

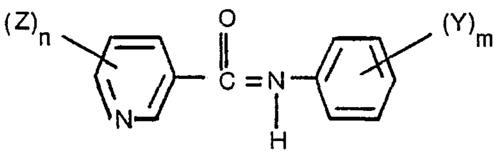

(III)

worin Y, Z, n und m die in Anspruch 1 angegebene Bedeutung aufweisen, mit Thionylchlorid.